# EUROPÄISCHE PATENTSCHRIFT

(11) **EP 3 229 760 B1**
(45) Veröffentlichungstag und Bekanntmachung des Hinweises auf die Patenterteilung: **30.09.2020**
(21) Anmeldenummer: 15787620.2
(22) Anmeldetag: 02.11.2015
(51) Int. Cl.: A61K 8/34, A61K 8/81, A61K 8/04, A61Q 5/06

(54) **KOSMETISCHES PRODUKT ENTHALTEND EIN POLARES LÖSUNGSMITTEL UND EINEN VERDICKER IN EINER VORRICHTUNG ZUR ENTSPANNUNGSVERDAMPFUNG**
COSMETIC PRODUCT CONTAINING A POLAR SOLVENT AND A THICKENER IN A FLASH EVAPORATION DEVICE
PRODUIT COSMÉTIQUE CONTENANT UN SOLVANT POLAIRE ET UN ÉPAISSISSANT DANS UN DISPOSITIF DE VAPORISATION ÉCLAIR

(30) Priorität: 10.12.2014 DE 102014225424
(43) Veröffentlichungstag der Anmeldung: 18.10.2017
(73) Patentinhaber: Henkel AG & Co. KGaA, 40589 Düsseldorf (DE)
(72) Erfinder: FÖRSTER, Thomas, 40591 Düsseldorf (DE); BAYERSDÖRFER, Rolf, 22589 Hamburg (DE); KNAPPE, Thorsten, 22869 Schenefeld (DE)
(86) Internationale Anmeldenummer: PCT/EP2015/075373
(87) Internationale Veröffentlichungsnummer: WO 2016/091460

(56) Entgegenhaltungen:
- EP-A1- 1 634 579
- WO-A1-2005/060926
- DE-T2- 69 107 225
- JP-A- 2007 319 234
- US-A- 3 372 840
- US-A1- 2002 074 349
- US-A1- 2002 079 377
- US-A1- 2004 065 683
- US-A1- 2012 201 774
- US-A1- 2013 018 333
- BASF: "Acrylic terpolymer products for hair-setting preparations with a strong, long-lasting effect (Luvimer)", TECHNICAL INFORMATION BASF,, 1. September 2000 (2000-09-01), Seiten 1-21, XP007921915,

## Beschreibung

Die Anmeldung betrifft das technische Fachgebiet der temporären Umformung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinhaltiger Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinhaltigen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Im Bereich der temporären Verformung keratinhaltiger Fasern hat insbesondere die Sprühapplikation entsprechender kosmetischer Zubereitungen eine große Bedeutung, wobei die Zubereitungen in der Regel als Pumpsprays oder Aerosolsprays appliziert werden. Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel. Aufgabe der vorliegenden Erfindung war es daher, spezifische haarkosmetische Zubereitungen zur temporären Verformung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens einen hohen Haltegrad, insbesondere einen hohen Langzeithaltegrad, und einen hohen Volumeneffekt zu realisieren. Es hat sich gezeigt, dass zur Lösung dieser Aufgabe aus der Vielzahl bekannten haarkosmetisch wirksamen Polymerzubereitungen insbesondere lösungsmittelhaltige Zubereitungen mit einer spezifischen Viskosität geeignet sind.

Ein erster Gegenstand der vorliegenden Erfindung ist somit ein kosmetisches Produkt, umfassend
a) eine kosmetische Zubereitung mit einer Viskosität von 20.000 bis 250.000 mPas, enthaltend bezogen auf ihr Gesamtgewicht
   a1) 80 bis 95 Gew.-% mindestens eines polaren Lösungsmittels;
   a2) 0,1 bis 5,0 Gew.-% mindestens eines Verdickers;
b) eine Vorrichtung zur Entspannungsverdampfung der kosmetischen Zubereitung a) mit
   b1) einem Behälter, welcher einen geschlossenen Innenraum definiert, in dem die kosmetische Zubereitung aufgenommen werden kann,
   b2) einem Ventil oder einem vergleichbar wirkenden Schließelement, um den wenigstens anteilsweise mit der kosmetischen Zubereitung a) befüllten Innenraum des Behälters zu verschließen und zu öffnen,
   b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
   b4) einer Düse, welche eine Zerstäubung der aus dem Behälter entweichenden kosmetischen Zubereitung a) ermöglicht,
c) einem Vorratsbehälter für die kosmetische Zubereitung a), aus welchem die kosmetische Zubereitung a) in den Behälter b1) gelangen kann, wobei
   - der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
   - der Vorratsbehälter mindestens das Zehnfache Volumen, vorzugsweise mindestens das Fünfzigfache Volumen des Behälters b1) aufweist,
   - der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Die Bestimmung der Viskosität erfolgt mittels eines Brookfield Viskosimeters Modell D220 (20°C, Spindel 6, 5 rpm).

Die kosmetische Zubereitung a) ist flüssig. Sie kann als Lösung oder Dispersion, beispielsweise als Emulsion oder Suspension vorliegen. Bevorzugte kosmetische Zubereitungen a) liegen in Form einer Lösung oder einer Suspension vor. Für die Anwendungseigenschaften kosmetischer Zubereitungen haben sich spezifische Viskositätswerte als besonders vorteilhaft erwiesen. Bevorzugte kosmetische Zubereitungen a) sind daher durch gekennzeichnet, dass ihre Viskosität 10.000 bis 50.0000 mPas, vorzugsweise 20.000 bis 250.000 mPas beträgt.

Die erfindungsgemäße kosmetische Zubereitung enthält als ersten wesentlichen Bestandteil 80 bis 95 Gew.-% mindestens eines polaren Lösungsmittels a1). Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol und Wasser erwiesen, welche aus diesem Grund als polare Lösungsmittel a1) bevorzugt werden.

Besonders bevorzugte polare Lösungsmittel a1) oder Lösungsmittelsysteme sind dadurch gekennzeichnet, dass
- der Gewichtsanteil von Wasser und Ethanol am Gesamtgewicht des polaren Lösungsmittels a1) vorzugsweise mindestens 60 Gew.-%, bevorzugt mindestens 80 Gew.-%, besonders bevorzugt mindestens 90 Gew.-% und insbesondere mindestens 95 Gew.-% beträgt;
- das polare Lösungsmittel a1), bezogen auf sein Gesamtgewicht, mehr als 80 Gew.-%, vorzugsweise mehr als 88 Gew.-% und insbesondere mehr als 92 Gew.-% Wasser umfasst.

Eine spezifische Ausführungsform ist dadurch gekennzeichnet, dass das polare Lösungsmittel a1) Wasser und Ethanol umfasst und Gewichtsverhältnis von Wasser zu Ethanol 5:1 bis 1:5, bevorzugt 2:1 bis 1:2 und insbesondere 5:4 bis 4:5 beträgt.

Ein zweiter wesentlicher Bestandteil erfindungsgemäßer kosmetischer Zusammensetzungen ist der Verdicker a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Verdickers a2) am Gesamtgewicht der kosmetischen Zubereitung 0,1 bis 5,0 Gew.-% beträgt.

Bevorzugte Verdicker sind ausgewählt aus der Gruppe der polymeren organischen Verdicker. Die polymeren organischen Verdicker können vernetzt oder unvernetzt sein.

Eine erste Gruppe besonders bevorzugter Verdicker a2) enthalten mindestens eine Struktureinheit ausgewählt aus mindestens einer Struktureinheit der Formel (I) oder deren Salzformen oder mindestens einer Struktureinheit -II) oder deren Salzformen, 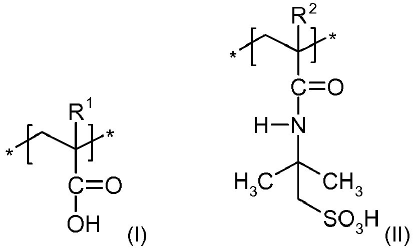 worin R¹ und R² unabhängig voneinander für ein Wasserstoffatom oder eine Methylgruppe stehen.

Gemäß obiger Formeln und allen folgenden Formeln steht eine chemische Bindung, die mit dem Symbol * gekennzeichnet ist, für eine freie Valenz des entsprechenden Strukturfragments.

Besonders bevorzugte anionische, verdickend wirkende Polymere enthalten mindestens eine Struktureinheit der Formel (I). Acrylsäurehomopolymere bilden eine erste Gruppe besonders bevorzugter Verdicker a2).

Besonders bevorzugte Verdicker sind
- Polyacrylsäuren mit der INCI-Bezeichnung Carbomer, wie sie beispielsweise von der Firma 3V Sigma unter dem Handelsnamen Synthalen® K oder von der Firma Lubrizol unter dem Handelsnamen Carbopol vertrieben werden.

Eine zweite besonders bevorzugte Gruppe von Verdickern a2) bilden die polymeren, anionischen, amphiphilen Verdicker. Entsprechende Verdicker umfassen bevorzugt mindestens eine Struktureinheit der Formel (III) und mindestens eine Struktureinheit der Formel (IV), 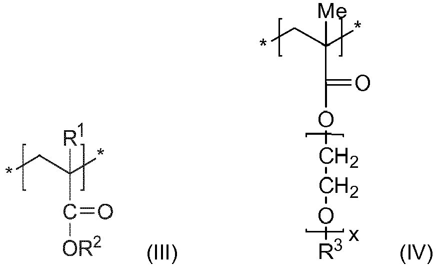 worin
R¹ steht für ein Wasserstoffatom oder eine Methylgruppe,
R² steht für ein Wasserstoffatom oder eine (C₁ bis C₆)-Alkylgruppe
R³ steht für eine (C₈ bis C₃₀)-Alkylgruppe,
M⁺ steht für ein physiologisch verträgliches Kation und
x steht für eine ganze Zahl von 0 bis 35.

Bevorzugt sind dabei insbesondere die Verdicker mit den INCI-Bezeichnungen Acrylates/Beheneth-25 Methacrylate Copolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-20 Acrylate Copolymer, Acrylates/Palmeth-25 Acrylate Copolymer, Acrylates/Palmeth-25 Itaconate Copolymer und Acrylates/Steareth-50 Acrylate Copolymer.

Besonders bevorzugte Verdicker sind
- Verdicker mit der INCI-Bezeichnung Acrylates/Steareth-20 Methacrylate Copolymer, wie sie beispielsweise unter der Handelsnamen Aculyn® 22 von der Firma Rohm&Haas vertrieben werden;
- Verdicker mit der INCI-Bezeichnung Acrylates/Steareth-20 Methacrylate Crosspolymer, wie sie beispielsweise unter der Handelsnamen Aculyn® 88 von der Firma Rohm&Haas vertrieben werden;
- Verdicker mit der INCI-Bezeichnung Acrylates/Steareth-20 Itaconate Copolymer, wie sie beispielsweise unter dem Handelsnamen Structure 2001 von der Firma National Starch vertrieben werden.

Weitere polymere, anionische, amphiphile Verdicker zeichnen sich durch langkettige Alkylsubstituenten aus. Zu dieser Gruppe zählen beispielsweise die Verbindungen mit den INCI-Bezeichnungen Acrylates/Stearyl Methacrylate Copolymer, Acrylates/Vinyl Isodecanoate Crosspolymer.

Besonders bevorzugte Verdicker sind
- Verdicker mit der INCI-Bezeichnung Acrylates/C₁₀₋₃₀ Alkyl Acrylate Crosspolymer, wie sie beispielsweise unter dem Handelsnamen Carbopol Ultrez 21 von der Firma Lubrizol vertreiben werden.

Weitere Verdicker a2) können beispielsweise ausgewählt werden unter den unter folgenden INCI-Bezeichnungen bekannten polymeren Verdickungsmitteln: Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium AcryloyldimethyltaurateNinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (po = 1,000 bar), entlastet werden kann, wodurch mindestens teilweise eine Verdampfung der kosmetischen Zubereitung a) erreicht wird.
Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung wird die kosmetische Zubereitung a) einer Düse zugeführt, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher Teil des Produkts. Der spezifische Düsentyp oder die spezifische Düsengestaltung wird in Abhängigkeit von den jeweiligen Sprühnebeleigenschaften gezielt festgelegt.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein Beispiel für einen bevorzugten weiteren Wirk- und Hilfsstoff sind die filmbildenden Polymere a3), deren Einsatz in den erfindungsgemäßen kosmetischen Mitteln besonders bevorzugt ist. Als filmbildende Polymere a3) eignen sich permanent als auch temporär kationische, anionische, nichtionische oder amphotere Polymere. Diese filmbildenden Polymere können synthetischen oder natürlichen Ursprungs sein. Bevorzugte kosmetische Zubereitung a) enthalten bezogen auf ihr Gesamtgewicht 0,1 bis 20 Gew.-%, vorzugsweise 0,5 bis 15 Gew.-%, und insbesondere 1,0 bis 10 Gew.-% eines filmbildenden Polymers a3). Dieses Polymer a3) ist von dem Polymer a2) verschieden.

Beispiele für gebräuchliche filmbildende Polymere a3) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Besonders bevorzugt sind kosmetische Produkte, dadurch gekennzeichnet, dass das filmbildende Polymer a3) ausgewählt ist aus der Gruppe der anionischen Polymere, vorzugsweise aus der Gruppe der Copolymere von Acrylsäure und Methacrylsäure.

Ein besonders bevorzugtes anionisches Acrylatcopolymer a3) ist zumindest aus folgenden Monomereinheiten aufgebaut: mindestens einer (Meth)Acrylsäureeinheit (V), mindestens einer (Meth)Acrylsäurealkylestereinheit (VI) und mindestens einer (Meth)Acrylsäurehydroxyalkylestereinheit (VII). Dieses bevorzugte Copolymer a3) kann erfindungsgemäß aus weiteren Monomereinheiten aufgebaut sein. Gemäß Ausführungsformen der Erfindung ist das Copolymer a3) aber nur aus den Einheiten (V), (VI) und (VII) aufgebaut, d. h. es besteht aus von diesen Monomereinheiten abgeleiteten Einheiten.

Die mindestens eine Methacrylsäureeinheit (V) kann eine Methacrylsäure- oder Acrylsäureeinheit sein.

Der Alkylrest der (Meth)Acrylsäurealkylestereinheit (VI) ist bevorzugt ein C1-C8-Alkylrest, der linear oder verzweigt sein kann. Beispiele für Alkylreste sind Methyl, Ethyl, Propyl, Isopropyl, 1-Butyl, 2-Butyl, iso-Butyl,tert-Butyl, lineares oder verzweigtes Pentyl, lineares oder verzweigtes Hexyl, lineares oder verzweigtes Heptyl und lineares oder verzweigtes Octyl. Bevorzugter ist die Alkylgruppe eine C1- bis C5-Alkylgruppe. Gemäß einer Ausführungsform der Erfindung sind zwei oder mehr (Meth)Acrylsäurealkylestereinheiten (VI) enthalten, die sich hinsichtlich der Kohlenstoffzahl der Alkylgruppe unterscheiden. Beispielsweise sind eine C1-C3-Alkylmethacrylateinheit und eine C2-C5-Alkylacrylateinheit enthalten.

Der Hydroxyalkylrest der (Meth)Acrylsäurehydroxyalkylestereinheit (VII) kann ein Hydroxy-C1-C10-Alkylrest sein, bevorzugt ein Hydroxy-C2-C5-Alkylrest. In einer bevorzugten Ausführungsform ist die (Meth)Acrylsäurehydroxyalkylestereinheit (VII) (Meth)Acrylsäurehydroxyethylester.

Der Anteil der Einheiten (V), (VI) und (VII) in dem Acrylatharz a3) kann in weiten Grenzen variieren. Der Anteil der Einheit (1) in dem Acrylatcopolymer ist bevorzugt 2 bis 50 Gew.-%, bevorzugter 5 bis 30 Gew.-%. Der Anteil der Einheit (2) in dem Acrylatcopolymer ist bevorzugt 5 bis 95 Gew.-%, bevorzugter 45 bis 90 Gew.-%. Der Anteil der Einheit (3) in dem Acrylatcopolymer ist bevorzugt 2 bis 70 Gew.-%, bevorzugter 5 bis 30 Gew.-%.

Geeignete anionische Acrylatcopolymere a3) sind im Handel unter der INCI-Bezeichnung Acrylates / Hydroxyesters Acrylates Copolymer erhältlich. Am bevorzugtesten ist das anionische Acrylatcopolymer (a) Acudyne® 1000 von The Dow Chemical Company.

Ein weiteres bevorzugtes anionisches Acrylatcopolymer a3) umfasst Struktureinheiten der Formel (VIII) worin R¹ für eine Methylgruppe und R² für eine Methylgruppe steht, und Struktureinheiten der Formel (VIII) worin R¹ für ein Wasserstoffatom und R² für eine Butylgruppe (insbesondere für eine n-Butylgruppe) steht, und Struktureinheiten der Formel (IX) worin R³ für eine Methylgruppe und R⁴ für eine 2-Hydroxyethylgruppe steht und Struktureinheiten der Formel (X) worin R⁷ für eine Methylgruppe steht mindestens eine Struktureinheit der Formel (XI) worin R⁵ und R⁶ unabhängig voneinander für ein Wasserstoffatom oder eine (C₁ bis C₆)-Alkylgruppe, vorzugsweise für Wasserstoff stehen

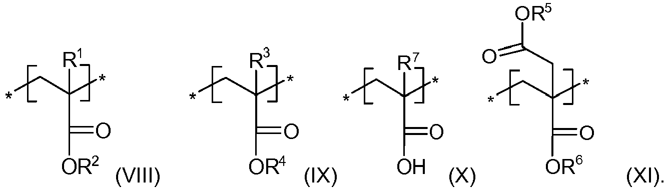

Ein besonders bevorzugtes Polymer trägt die INCI-Nomenklatur Acrylates / C1-2 Succinates / Hydroxyacrylates Copolymer. Es kann beispielsweise von der Firma Dow unter dem Handelsnamen Acudyne LT-120 erworben werden (INCI-Nomenklatur: Acrylates / C1-2 Succinates / Hydroxyacrylates Copolymer).

Eine zweite Gruppe mit Vorzug in der kosmetischen Zubereitung eingesetzten Polymere a3) sind die Vinylpyrrolidon Homo- oder Copolymere B. Mit besonderem Vorzug eingesetzte Polymere B sind beispielsweise:
- Polyvinylpyrrolidone, wie sie beispielsweise unter der Bezeichnung Luviskol® (BASF) vertrieben werden,
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.

Aufgrund ihrer kosmetischen Wirkung in Kombination mit den Copolymeren a2) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer). Bevorzugte kosmetisches Produkte sind dadurch gekennzeichnet, dass das filmbildende Polymer a3) ausgewählt ist aus der Gruppe der nichtionischen Polymere, vorzugsweise aus der Gruppe der Polyvinylpyrrolidone und Vinylpyrrolidon/Vinylacetat-Copolymere, vorzugsweise der Polyvinylpyrrolidone.

Eine dritte Gruppe besonders bevorzugter filmbildender Polymere a3) bildet die Gruppe der Copolymere des Methacryloylethyl-N,N-dimethylaminoxids.

In einer besonders geeigneten Ausführungsform enthält die erfindungsgemäße kosmetische Zubereitung mindestens ein amphoteres festigendes Polymer, das gebildet ist aus
- mindestens einem Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester,
- mindestens einem zweiten vom ersten verschiedenen Monomer ausgewählt aus Acrylsäurestearylester und Methacrylsäurestearylester, und
- Methacryloylethylaminoxid, insbesondere Methacryloylethyl-N,N-dimethylaminoxid.

Diese Copolymere tragen beispielsweise die INCI-Bezeichnung Acrylates/Stearyl Acrylate/Ethylamine Oxide Methacrylate Copolymer und sind unter dem Handelsnamen Diaformer Z-632 von der Firma Clariant erhältlich.

In einer geeigneten Ausführungsform enthält das erfindungsgemäße Mittel mindestens ein amphoteres festigendes Polymer, das gebildet ist aus
- mindestens einem Monomer ausgewählt aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester,
- mindestens einem zweiten vom ersten verschiedenen Monomer ausgewählt aus Acrylsäurelaurylester und Methacrylsäurelaurylester,
- mindestens einem dritten, vom ersten und zweiten verschiedenen Monomer ausgewählt aus Acrylsäurestearylester und Methacrylsäurestearylester, und
- Methacryloylethylaminoxid, insbesondere Methacryloylethyl-N,N-dimethylaminoxid.

Entsprechende Copolymere mit der INCI-Bezeichnung Acrylates/Lauryl Acrylates/Stearyl Acrylate/Ethylamine Oxide Methacrylate Copolymer sind beispielsweise unter den Handelsnamen Diaformer Z-712N und Diaformer Z-731N von der Firma Clariant erhältlich.

Eine vierte Gruppe besonders bevorzugter filmbildender Polymere a3) umfasst Copolymere a3), welche auf die Monomere i) N-tert-Octylacrylamid, ii) Acrylsäure, iii) tert.-Butylaminoethylmethacrylat sowie gegebenenfalls weitere Monomere zurückführbar sind.

Bevorzugte Copolymere a3) bestehen bevorzugt zu mindestens 90 Gew.-%, vorzugsweise zu mindestens 95 Gew.-% und insbesondere zu mindestens 97 Gew.-% aus den Monomeren N-tert-Octylacrylamid, Acrylsäure und tert.-Butylaminoethylmethacrylat. Besonders bevorzugte Copolymere a3) wurden ausschließlich aus den Monomeren N-tert-Octylacrylamid, Acrylsäure und tert.-Butylaminoethylmethacrylat erhalten.

Besonders bevorzugt sind Copolymere a3) aus den Monomeren i) N-tert-Octylacrylamid, ii) Acrylsäure, iii) tert.-Butylaminoethylmethacrylat, iv) Methylmethacrylat und v) Hydroxypropylmethacrylat.
Die zuvor beschriebenen Copolymere a3) werden beispielsweise unter der Bezeichnung Amphomer® (INCI-Bezeichnung: Octylacrylamide / Acrylates / Butylaminoethyl Methacrylate Copolymer; CAS Nummer 70801-07-9) von der Firma National Starch vertrieben.

Zur Verbesserung der Herstellbarkeit, Applizierbarkeit und kosmetischen Wirkung enthält die kosmetische Zubereitung a) vorzugsweise nichtionisches Tensid a4), wobei besonders bevorzugte kosmetische Zubereitungen a) dadurch gekennzeichnet sind, dass sie bezogen auf ihr Gesamtgewicht 0,02 bis 4,0 Gew.-%, vorzugsweise 0,05 bis 2,0 Gew.-% und insbesondere 0,1 bis 1,0 Gew.-% nichtionisches Tensid a4) enthalten.

Bevorzugte nichtionische Tenside sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil. Erfindungsgemäß besonders bevorzugt sind nichtionische Tenside ausgewählt aus der Gruppe der PEG-Derivate von hydriertem Ricinusöl, besonders bevorzugt aus der Gruppe PEG-40 Hydrogenated Castor Oil und PEG-60 Hydrogenated Castor Oil, insbesondere PEG-40 Hydrogenated Castor Oil enthält.

Ein weiterer bevorzugter Bestandteil erfindungsgemäßer kosmetischer Zubereitungen a) sind die kationischen Tenside a5). Bevorzugte kationische Tenside a5) sind ausgewählt unter quartären Ammoniumverbindungen, Esterquats und Amidoaminen. Die kationischen Tenside sind in der kosmetischen Zubereitung a), bezogen auf deren Gesamtgewicht, in Mengen von 0,05 bis 3,0 Gew.-%, vorzugsweise 0,1 bis 2,0 Gew.-%, und insbesondere 0,2 bis 1,0 Gew.-% enthalten. Kationsiche Tenside a5) aus der Gruppe der quartären Ammoniumverbindungen sind besonders bevorzugt.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Ganz besonders bevorzugte erfindungsgemäße kosmetische Zubereitungen a) sind dadurch gekennzeichnet, dass sie bezogen auf ihr Gesamtgewicht 0,05 bis 3,0 Gew.-%, vorzugsweise 0,1 bis 2,0 Gew.-%, und insbesondere 0,2 bis 1,0 Gew.-% (C₁₂ bis C₁₈)-Alkyltrimethylammoniumsalz(e) enthalten.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 41 | Formel 42 | Formel 43 | Formel 44 | Formel 45 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| optionale Additive | add 100 | add 100 | add 100 | add 100 | add 100 |

| | Formel 46 | Formel 47 | Formel 48 | Formel 49 | Formel 50 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 51 | Formel 52 | Formel 53 | Formel 54 | Formel 55 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 56 | Formel 57 | Formel 58 | Formel 59 | Formel 60 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| filmbildendes Polymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 61 | Formel 62 | Formel 63 | Formel 64 | Formel 65 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 66 | Formel 67 | Formel 68 | Formel 69 | Formel 70 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 71 | Formel 72 | Formel 73 | Formel 74 | Formel 75 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 76 | Formel 77 | Formel 78 | Formel 79 | Formel 80 |
|---|---|---|---|---|---|
| polares Lösungsmittel a1) | 45 bis 99 | 70 bis 98 | 80 bis 95 | 90 | 90 |
| Verdicker a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 81 | Formel 82 | Formel 83 | Formel 84 | Formel 85 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 86 | Formel 87 | Formel 88 | Formel 89 | Formel 90 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 91 | Formel 91 | Formel 93 | Formel 94 | Formel 95 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 96 | Formel 97 | Formel 98 | Formel 99 | Formel 100 |
|---|---|---|---|---|---|
| polares Lösungsmittel a1) | 45 bis 99 | 70 bis 98 | 80 bis 95 | 90 | 90 |
| Polyacrylsäure a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 101 | Formel 102 | Formel 103 | Formel 104 | Formel 105 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 106 | Formel 107 | Formel 108 | Formel 109 | Formel 110 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 111 | Formel 112 | Formel 113 | Formel 114 | Formel 115 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 116 | Formel 117 | Formel 118 | Formel 119 | Formel 120 |
|---|---|---|---|---|---|
| polares Lösungsmittel a1) | 45 bis 99 | 70 bis 98 | 80 bis 95 | 90 | 90 |
| polymerer, anionischer, amphiphiler Verdicker a2) | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| nichtionisches Tensid a4) | 0,02 bis 4,0 | 0,05 bis 2,0 | 0,1 bis 1,0 | 0,6 | 0,5 |
| optionale Additive | add 100 | add 100 | add 100 | add 100 | add 100 |

| | Formel 121 | Formel 122 | Formel 123 | Formel 124 | Formel 125 |
|---|---|---|---|---|---|
| polares Lösungsmittel a1) | 45 bis 99 | 70 bis 98 | 80 bis 95 | 90 | 90 |
| Acrylates/Steareth-20 Methacrylate Copolymer | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 126 | Formel 127 | Formel 128 | Formel 129 | Formel 130 |
|---|---|---|---|---|---|
| polares Lösungsmittel a1) | 45 bis 99 | 70 bis 98 | 80 bis 95 | 90 | 90 |
| Acrylates/Steareth-20 Methacrylate Copolymer | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 131 | Formel 132 | Formel 133 | Formel 134 | Formel 135 |
|---|---|---|---|---|---|
| polares Lösungsmittel a1) | 45 bis 99 | 70 bis 98 | 80 bis 95 | 90 | 90 |
| Acrylates/Steareth-20 Methacrylate Crosspolymer | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 136 | Formel 137 | Formel 138 | Formel 139 | Formel 140 |
|---|---|---|---|---|---|
| polares Lösungsmittel a1) | 45 bis 99 | 70 bis 98 | 80 bis 95 | 90 | 90 |
| Acrylates/Steareth-20 Methacrylate Crosspolymer | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 141 | Formel 142 | Formel 143 | Formel 144 | Formel 145 |
|---|---|---|---|---|---|
| polares Lösungsmittel a1) | 45 bis 99 | 70 bis 98 | 80 bis 95 | 90 | 90 |
| Acrylates/Steareth-20 Itaconate Copolymer | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 146 | Formel 147 | Formel 148 | Formel 149 | Formel 150 |
|---|---|---|---|---|---|
| polares Lösungsmittel a1) | 45 bis 99 | 70 bis 98 | 80 bis 95 | 90 | 90 |
| Acrylates/Steareth-20 Itaconate Copolymer | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 151 | Formel 152 | Formel 153 | Formel 154 | Formel 155 |
|---|---|---|---|---|---|
| polares Lösungsmittel a1) | 45 bis 99 | 70 bis 98 | 80 bis 95 | 90 | 90 |
| Acrylates/C₁₀₋₃₀ Alkyl Acrylate Crosspolymer | 0,01 bis 10 | 0,05 bis 8,0 | 0,1 bis 5,0 | 0,3 | 1,5 |
| Poylvinylpyrrolidon a3) | 0,1 bis 20 | 0,5 bis 15 | 1,0 bis 10 | 2,5 | 1,6 |
| optionale Additive | add 100 | add 100 | add 100 | add 100 | add 100 |

| | Formel 156 | Formel 157 | Formel 158 | Formel 159 | Formel 160 |
|---|---|---|---|---|---|
| polares Lösungsmittel a1) | 45 bis 99 | 70 bis 98 | 80 bis 95 | 90 | 90 |
| Acrylates/C₁₀₋₃₀ Alkyl Acrylate Crosspolymer | 0,01 bis 10 | 0,05 bis 8,0 | 0,1 bis 5,0 | 0,3 | 1,5 |
| Vinylpyrrolidon/Vinylacetat-Copolymer a3) | 0,1 bis 20 | 0,5 bis 15 | 1,0 bis 10 | 2,5 | 1,6 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a5) nur geringe Mengen weiterer Wirk- und Hilfsstoffe. Kosmetische Zubereitungen, dadurch gekennzeichnet, dass die kosmetische Zubereitung bezogen auf ihr Gesamtgewicht, zu mindestens 80 Gew.-%, vorzugsweise mindestens 90 Gew.-% und insbesondere mindestens 97 Gew.-% aus den Bestanteilen a1), a2), a3), a4) und, sofern vorhanden, a5) besteht, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 80 Gew.-%, vorzugsweise mindestens 90 Gew.-% und insbesondere mindestens 97 Gew.-% aus den Bestanteilen a1), a2) und a3).

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) mit einer Viskosität von 20.000.bis 250.000 mPas enthaltend, bezogen auf ihr Gesamtgewicht,
a1) 80 bis 95 Gew.-% mindestens eines polaren Lösungsmittels;
a2) 0,1 bis 5,0 Gew.-% mindestens eines Verdickers;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen.

Gegenstand der vorliegenden Anmeldung ist zudem die Verwendung eines erfindungsgemäßen Produktes zur Beaufschlagung keratinhaltiger Fasern, insbesondere menschlicher Haare, mit einer kosmetischen Zubereitung a) bzw. zur temporären Verformung keratinhaltiger Fasern, insbesondere menschlicher Haare.

Ein Verfahren zur temporären Verformung keratinhaltiger Fasern, insbesondere menschlicher Haare, bei welchem die keratinhaltigen Fasern mittels einer Vorrichtung zur Entspannungsverdampfung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen;
mit einer kosmetischen Zubereitung a) mit einer Viskosität von 20.000 bis 250.000 mPas, enthaltend bezogen auf ihr Gesamtgewicht
a1) 80 bis 95 Gew.-% mindestens eines polaren Lösungsmittels;
a2) 0,1 bis 5,0 Gew.-% mindestens eines Verdickers; beaufschlagt werden.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindungsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung mit einer Viskosität von 20.000 bis 250.000 mPas, enthaltend bezogen auf ihr Gesamtgewicht
a1) 80 bis 95 Gew.-% mindestens eines polaren Lösungsmittels;
a2) 0,1 bis 5,0 Gew.-% mindestens eines Verdickers;
b) eine Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
c) einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

2. Kosmetisches Produkt nach Anspruch 1, **dadurch gekennzeichnet, dass** der Verdicker a2) ausgewählt ist aus der Gruppe der polymeren organischen Verdicker.

3. Kosmetisches Produkt nach einem der vorherigen Ansprüche, **dadurch gekennzeichnet, dass** die kosmetische Zubereitung a) bezogen auf ihr Gesamtgewicht 0,1 bis 20 Gew.-%, vorzugsweise 0,5 bis 15 Gew.-%, und insbesondere 1,0 bis 10 Gew.-% eines filmbildenden Polymers a3) enthält.

4. Verwendung einer kosmetischen Zubereitung a) mit einer Viskosität von 20.000 bis 250.000 mPas enthaltend, bezogen auf ihr Gesamtgewicht,
a1) 80 bis 95 Gew.-% mindestens eines polaren Lösungsmittels;
a2) 0,1 bis 5,0 Gew.-% mindestens eines Verdickers;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen.

5. Verwendung eines Produkts nach einem der Ansprüche 1 bis 3 zur Beaufschlagung keratinhaltiger Fasern, insbesondere menschlicher Haare, mit einer kosmetischen Zubereitung a).

6. Verwendung eines Produkts nach einem der Ansprüche 1 bis 3 zur temporären Verformung keratinhaltiger Fasern, insbesondere menschlicher Haare.

7. Verfahren zur temporären Verformung keratinhaltiger Fasern, insbesondere menschlicher Haare, bei welchem die keratinhaltigen Fasern mittels einer Vorrichtung zur Entspannungsverdampfung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
einem Vorratsbehälter für die kosmetische Zubereitung a), aus welchem die kosmetische Zubereitung a) in den Behälter b1) gelangen kann, wobei
- der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann,
- der Vorratsbehälter mindestens das Zehnfache Volumen, vorzugsweise mindestens das Fünfzigfache Volumen des Behälters b1) aufweist,
- der Druck im Inneren des Vorratsbehälters dem Umgebungsdruck entspricht und das kosmetische Produkt kein Treibmittel umfasst,
wobei keine Pumpvorrichtung vorgesehen ist, die geeignet ist, die kosmetische Zubereitung a) ohne die Einwirkung der Vorrichtung zur Entspannungsverdampfung freizusetzen oder zu versprühen;
mit einer kosmetischen Zubereitung a) mit einer Viskosität von 20.000 bis 250.000 mPas, enthaltend bezogen auf ihr Gesamtgewicht
a1) 80 bis 95 Gew.-% mindestens eines polaren Lösungsmittels;
a2) 0,1 bis 5,0 Gew.-% mindestens eines Verdickers;
beaufschlagt werden.

## Claims

1. A cosmetic product, comprising
a) a cosmetic preparation having a viscosity of from 20,000 to 250,000 mPas containing, based on the total weight thereof
a1) 80 to 95 wt.% of at least one polar solvent;
a2) 0.1 to 5.0 wt.% of at least one thickener;
b) a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which allows atomization of the cosmetic preparation a) escaping from the container,
c) a storage container for the cosmetic preparation a), from which the cosmetic preparation a) can enter the container b1), wherein
- the access between the storage container and the container b1) has a component for flow control, by means of which component the flow of the cosmetic preparation a) from the storage container into the container b1) can be interrupted,
- the storage container has at least ten times the volume, preferably at least fifty times the volume of the container b1),
- the pressure inside the storage container corresponds to the ambient pressure and the cosmetic product does not contain a propellant,
wherein the cosmetic product does not have a pumping device which is suitable for releasing or spraying the cosmetic preparation a) without the action of the device for flash evaporation.

2. The cosmetic product according to claim 1, **characterized in that** the thickener a2) is selected from the group of polymeric organic thickeners.

3. The cosmetic product according to one of the preceding claims, **characterized in that** the cosmetic preparation a) contains, based on the total weight thereof, 0.1 to 20 wt.%, preferably 0.5 to 15 wt.%, and in particular 1.0 to 10 wt.% of a film-forming polymer a3).

4. The use of a cosmetic preparation a) having a viscosity of from 20,000 to 250,000 mPas containing, based on the total weight thereof,
a1) 80 to 95 wt.% of at least one polar solvent;
b1) 0.1 to 5.0 wt.% of at least one thickener;
as a process material in a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which allows atomization of the cosmetic preparation a) escaping from the container,
a storage container for the cosmetic preparation a), from which the cosmetic preparation a) can enter the container b1), wherein
- the access between the storage container and the container b1) has a component for flow control, by means of which component the flow of the cosmetic preparation a) from the storage container into the container b1) can be interrupted,
- the storage container has at least ten times the volume, preferably at least fifty times the volume of the container b1),
- the pressure inside the storage container corresponds to the ambient pressure and the cosmetic product does not contain a propellant,
wherein there is no pumping device which is suitable for releasing or spraying the cosmetic preparation a) without the action of the device for flash evaporation.

5. The use of a product according to one of claims 1 to 3 for applying a cosmetic preparation a) to keratin-containing fibers, in particular human hair.

6. The use of a product according to one of claims 1 to 3 for temporarily shaping keratin-containing fibers, in particular human hair.

7. A method for temporarily shaping keratin-containing fibers, in particular human hair, in which, by means of a device for flash evaporation comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container, and releasing, under reduced pressure, the heated cosmetic preparation a) from the interior of the container into the surroundings,
b4) a nozzle which allows atomization of the cosmetic preparation a) escaping from the container,
a storage container for the cosmetic preparation a), from which the cosmetic preparation a) can enter the container b1), wherein
- the access between the storage container and the container b1) has a component for flow control, by means of which component the flow of the cosmetic preparation a) from the storage container into the container b1) can be interrupted,
- the storage container has at least ten times the volume, preferably at least fifty times the volume of the container b1),
- the pressure inside the storage container corresponds to the ambient pressure and the cosmetic product does not contain a propellant,
wherein there is no pumping device which is suitable for releasing or spraying the cosmetic preparation a) without the action of the device for flash evaporation;
a cosmetic preparation a) having a viscosity of from 20,000 to 250,000 mPas containing, based on the total weight thereof
a1) 80 to 95 wt.% of at least one polar solvent;
a2) 0.1 to 5.0 wt.% of at least one thickener;
is applied to the keratin-containing fibers.

## Revendications

1. Produit cosmétique, comprenant
a) une préparation cosmétique ayant une viscosité comprise entre 20 000 et 250 000 MPa, la préparation contenant, par rapport à son poids total,
a1) 80 à 95 % en poids d'au moins un solvant polaire ;
a2) 0,1 à 5,0 % en poids d'au moins un épaississant ;
b) un dispositif de vaporisation éclair de la préparation cosmétique a), le dispositif comportant
b1) un récipient définissant un espace intérieur fermé dans lequel la préparation cosmétique peut être reçue,
b2) une soupape ou un moyen de fermeture à action comparable permettant de fermer et d'ouvrir au moins partiellement l'espace intérieur du récipient rempli de la préparation cosmétique a),
b3) un dispositif de chauffage permettant de chauffer la préparation cosmétique a) présente dans l'espace intérieur fermé du récipient par augmentation de la pression, ainsi que de vaporiser la préparation cosmétique chauffée a) dans l'environnement, hors de l'espace intérieur du récipient, par réduction de la pression,
b4) une buse permettant une atomisation de la préparation cosmétique a) s'échappant du récipient,
c) un récipient de stockage destiné à la préparation cosmétique a), depuis lequel la préparation cosmétique a) peut pénétrer dans le récipient b1),
- l'accès entre le récipient de stockage et le récipient b1) présentant un élément de régulation de flux au moyen duquel le flux de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant pas d'agent gonflant,
le produit cosmétique ne présentant pas de dispositif de pompage approprié pour libérer ou pulvériser la préparation cosmétique a) sans l'action du dispositif de vaporisation éclair.

2. Produit cosmétique selon la revendication 1, **caractérisé en ce que** l'épaississant a2) est choisi dans le groupe des épaississants organiques polymères.

3. Produit cosmétique selon l'une des revendications précédentes, **caractérisé en ce que** la préparation cosmétique a) contient, par rapport à son poids total, 0,1 à 20 % en poids, de préférence 0,5 à 15 % en poids, plus préférentiellement 1,0 à 10 % en poids d'un polymère filmogène a3).

4. Utilisation d'une préparation cosmétique a) ayant une viscosité comprise entre 20 000 et 250 000 MPa, la préparation contenant, par rapport à son poids total,
a1) 80 à 95 % en poids d'au moins un solvant polaire ;
b1) 0,1 à 5,0 % en poids d'au moins un épaississant ;
comme produit de traitement dans un dispositif de vaporisation éclair de la préparation cosmétique a), le dispositif comportant
b1) un récipient définissant un espace intérieur fermé dans lequel la préparation cosmétique peut être reçue,
b2) une soupape ou un moyen de fermeture à action comparable permettant de fermer et d'ouvrir au moins partiellement l'espace intérieur du récipient rempli de la préparation cosmétique a),
b3) un dispositif de chauffage permettant de chauffer la préparation cosmétique a) présente dans l'espace intérieur fermé du récipient par augmentation de la pression, ainsi que de vaporiser la préparation cosmétique chauffée a) dans l'environnement, hors de l'espace intérieur du récipient, par réduction de la pression,
b4) une buse permettant une atomisation de la préparation cosmétique a) s'échappant du récipient,
un récipient de stockage destiné à la préparation cosmétique a), depuis lequel la préparation cosmétique a) peut pénétrer dans le récipient b1),
- l'accès entre le récipient de stockage et le récipient b1) présentant un élément de régulation de flux au moyen duquel le flux de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant pas d'agent gonflant,
aucun dispositif de pompage n'étant prévu, qui soit adapté pour libérer ou pulvériser la préparation cosmétique a) sans l'action du dispositif de vaporisation éclair.

5. Utilisation d'un produit selon l'une des revendications 1 à 3, pour exposer des fibres kératiniques, en particulier des cheveux humains, à une préparation cosmétique a).

6. Utilisation d'un produit selon l'une des revendications 1 à 3, pour déformer temporairement des fibres kératiniques, en particulier des cheveux humains.

7. Procédé de déformation temporaire de fibres kératiniques, en particulier de cheveux humains, selon lequel les fibres kératiniques sont exposées au moyen d'un dispositif de vaporisation éclair comportant
b1) un récipient définissant un espace intérieur fermé dans lequel la préparation cosmétique peut être reçue,
b2) une soupape ou un moyen de fermeture à action comparable permettant de fermer et d'ouvrir au moins partiellement l'espace intérieur du récipient rempli de la préparation cosmétique a),
b3) un dispositif de chauffage permettant de chauffer la préparation cosmétique a) présente dans l'espace intérieur fermé du récipient par augmentation de la pression, ainsi que de vaporiser la préparation cosmétique chauffée a) dans l'environnement, hors de l'espace intérieur du récipient chauffée, par réduction de la pression,
b4) une buse permettant une atomisation de la préparation cosmétique a) s'échappant du récipient,
un récipient de stockage destiné à la préparation cosmétique a), depuis lequel la préparation cosmétique a) peut pénétrer dans le récipient b1),
- l'accès entre le récipient de stockage et le récipient b1) présentant un élément de régulation de flux au moyen duquel le flux de la préparation cosmétique a) du récipient de stockage vers le récipient b1) peut être interrompu,
- le récipient de stockage présentant au moins dix fois le volume, de préférence au moins cinquante fois le volume du récipient b1),
- la pression à l'intérieur du récipient de stockage correspondant à la pression ambiante et le produit cosmétique ne comprenant pas d'agent gonflant,
aucun dispositif de pompage n'étant prévu, qui soit adapté pour libérer ou pulvériser la préparation cosmétique a) sans l'action du dispositif de vaporisation éclair ;
comportant une préparation cosmétique a) ayant une viscosité comprise entre 20 000 et 250 000 MPa, la préparation contenant, par rapport à son poids total,
a1) 80 à 95 % en poids d'au moins un solvant polaire ;
a2) 0,1 à 5,0 % en poids d'au moins un épaississant.
